# EUROPÄISCHE PATENTSCHRIFT

(11) **EP 1 374 932 B1**
(45) Veröffentlichungstag und Bekanntmachung des Hinweises auf die Patenterteilung: **26.04.2006**
(21) Anmeldenummer: 03013542.0
(22) Anmeldetag: 13.06.2003
(51) Int. Cl.: A61M 5/142, A61M 5/145

(54) **Spritzenpumpe**
Syringe pump
Pompe-seringue

(30) Priorität: 20.06.2002 DE 20209581 U
(43) Veröffentlichungstag der Anmeldung: 02.01.2004
(73) Patentinhaber: B. BRAUN MELSUNGEN AG, 34212 Melsungen (DE)
(72) Erfinder: Gerlach, Hans-Josef, 34431 Marsberg (DE); Sippel, Martin, Dr., 34212 Melsungen (DE)
(74) Vertreter: Selting, Günther

(56) Entgegenhaltungen:
- WO-A-00/47254
- GB-A- 621 208
- GB-A- 2 094 628
- US-A- 5 377 725
- US-A- 5 487 738
- US-A- 5 498 243

## Beschreibung

Die Erfindung betrifft eine Spritzenpumpe mit einem Gehäuse, das einen von einem Deckel mit Fenster verschließbaren Raum für eine einzulegende Spritze aufweist, einem relativ zu dem Gehäuse linear bewegbaren Antriebskopf zum Bewegen des Spritzenkolbens der Spritze, und einem Bedienfeld.

In der modernen Intensivmedizin kommen vermehrt Infusionsgeräte in großer Anzahl zum Einsatz. Ein einziger Intensivplatz kann bis zu zwanzig Infusionspumpen aufweisen. Dies erfordert eine platzsparende Gestaltung der Infusionspumpen. Diese müssen daher kleinformatig ausgebildet sein.

Der Oberbegriff des Patentanspruchs 1 geht aus von einer Spritzenpumpe gemäß GB-A-2094628. Diese Spritzenpumpe weist ein Gehäuse auf mit einem Raum, der durch einen Deckel hindurch zugänglich ist, und in dem eine Spritze abgestützt wird. Die Kolbenstange der Spritze wird an einem motorisch bewegbaren Antriebskopf zum Bewegen des Spritzenkolbens befestigt. Der Deckel enthält ein Fenster, durch das hindurch eine an dem Spritzenzylinder angebrachte Skala sichtbar ist. Diese Art der Befestigung der Spritze zwischen einem das eine Ende des Spritzenzylinders abstützenden Halter und einem an dem gegenüber liegenden Ende der Kolbenstange angreifenden Antriebskopf, ist in der Regel nur bei solchen Spitzenpumpen anwendbar, die für eine vorgegebene Spritzengröße oder einen engen Bereich von Spritzengrößen bestimmt sind.

US-A-5377725 beschreibt eine optische Vergrößerungsvorrichtung zur Erleichterung des Ablesens einer Spritze. Die Vergrößerungsvorrichtung weist ein Gehäuse auf, in das die Spritze seitlich eingeführt wird. In der Oberseite des Gehäuses befindet sich eine Vergrößerungslinse. Das Gehäuse enthält eine die Spritze abstützende Spritzenmulde.

Der Erfindung liegt die Aufgabe zugrunde, eine Spritzenpumpe zu schaffen, die trotz Kleinformatigkeit ein gutes Ablesen der in sie eingesetzten Spritze ermöglicht, wobei Spritzen unterschiedlicher Größen einsetzbar sind.

Die Lösung dieser Aufgabe erfolgt erfindungsgemäß mit den im Anspruch 1 angegebenen Merkmalen. Hiernach enthält der Raum eine die Spritze abstützende Spritzenmulde; der Deckel bedeckt die Spritzenmulde mindestens teilweise und ist aufklappbar und das Fenster enthält eine Lupe zur vergrößerten Darstellung der Spritzenoberfläche, die die Spritzenoberfläche unterschiedlich großer Spritzen, deren Durchmesser sich mindestens um den Faktor 2 unterscheidet, optisch vergrößert.

Ein Teil des Deckels ist als Lupe ausgebildet, um dem Anwender eine vergrößerte Abbildung des interessierenden Bereichs der Spritze verfügbar zu machen. Eine derartige optische Vergrößerung ist insbesondere dann hilfreich, wenn die Spritzenpumpe für Spritzen unterschiedlicher Formate geeignet ist. Bei kleinen Spritzen sind die Skala und die Zahlen notwendigerweise klein ausgeführt. Die Lupe bringt dann die Oberfläche der tief in der Spritzenmulde zurücküegenden Spritze in vergrößerter Form zum Auge des Betrachters. Dadurch wird vermieden, dass der Benutzer eine kleine Spritze zunächst mit dem Auge suchen und dann mühsam eine winzige Skala ablesen muss. Selbst bei größerem Abstand des Betrachters und aus ungünstigem Betrachtungswinkel ist eine kleine Spritze mühelos ablesbar.

Der Deckel kann zusätzlich eine Beleuchtung enthalten, die Licht in die Spritzenmulde leitet. Alternativ kann die Beleuchtungsvorrichtung an der Spritzenmulde vorgesehen sein. Die Unterstützung durch eine Beleuchtung ist besonders in abgedunkelter Umgebung hilfreich. Des weiteren kann durch eine Beleuchtung auch auf besondere Gerätezustände hingewiesen werden, um beispielsweise einen Alarm anzuzeigen. Hierfür können auch besondere Lichtfarben aktiviert werden.

Im Folgenden wird unter Bezugnahme auf die Zeichnungen ein Ausführungsbeispiel der Erfindung näher erläutert.

Es zeigen:
- Fig. 1: eine perspektivische Ansicht der Spritzenpumpe bei geschlossenem Deckel,

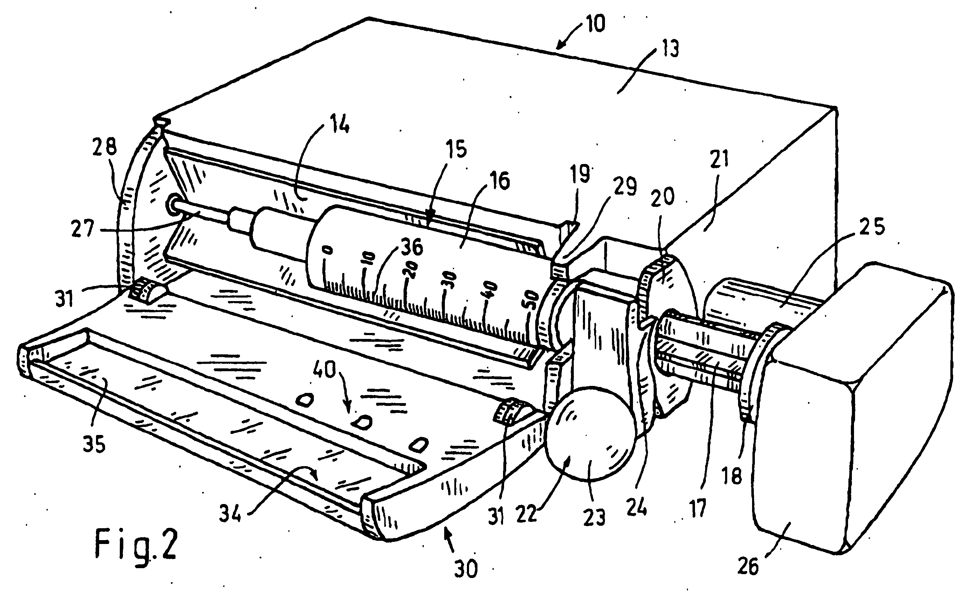

- Fig. 2: die Spritzenpumpe bei geöffnetem Deckel,
- Fig. 3: eine perspektivische Darstellung des Deckels, und
- Fig. 4: eine Schnittdarstellung zur Verdeutlichung der Eignung der Spritzenpumpe für unterschiedliche Spritzengrößen.

Die Spritzenpumpe weist ein Gehäuse 10 in Form eines flachen Kastens auf. An der Vorderseite des Gehäuses 10 befindet sich die horizontale Spritzenmulde 14, in die eine Spritze 15 eingelegt werden kann. Die Spritze 15 weist einen Spritzenzylinder 16 und einen darin befindlichen Kolben auf, der mit einer Kolbenstange 17 verbunden ist. Am Ende der Kolbenstange 17 befindet sich eine Kolbenplatte 18. Der Spritzenzylinder 16 hat an seinem proximalen Ende Spritzenflügel 20, die gegen ein Spritzenlager 21 des Gehäuses gesetzt werden, um die Spritze gegen axiales Verschieben zu sichern.

An dem Gehäuse 10 ist außerdem ein Spritzenbügel 22 vorgesehen, der manuell nach vorne herausziehbar ist und zu diesem Zweck einen Handgriff 23 aufweist. Der Spritzenbügel 22 wird von einer Feder in Richtung auf die Spritze 15 gezogen und er drückt somit die Spritze gegen die Spritzenmulde 14. Die Position des Spritzenbügels 22 wird abgetastet und elektronisch ausgewertet, um dadurch die Spritzengröße zu erkennen. Der Spritzenbügel 22 weist eine den Spritzenflügel 20 übergreifende Klinge 24 auf, die an der Kolbenstange 17 angreifen kann, um als Kolbenbremse zu wirken. Diese Klinge 24 kann gesteuert bewegt werden.

Zum Bewegen des Spritzenkolbens relativ zu dem Spritzenzylinder 16 ist an dem Gehäuse 10 eine Antriebsstange 25 vorgesehen, die aus einer Stirnseite des Gehäuses herausgefahren werden kann und an ihrem Ende ein Antriebskopf 26 trägt. Der Antriebskopf 26 enthält eine Greifvorrichtung, welche die Kolbenplatte 18 erfasst und festhält. Durch gesteuertes Bewegen der Antriebsstange 25 wird die Kolbenstange 17 vorgeschoben, um den Inhalt der Spritze 15 auszudrücken. An das distale Ende der Spritze 15 ist eine zu einem Patienten führende Infusionsleitung 27 angeschlossen.

Die Spritzenmulde 14 erstreckt sich an der Vorderseite des Gehäuses 10 zwischen der einen Stirnwand 28 und einer Zwischenwand 29. Die Spritzenmulde 14 wird durch einen Deckel 30 verschlossen, der mit Scharniergelenken 31 an der unteren Vorderkante des Gehäuses angelenkt ist. Der Deckel 30 legt sich im geschlossenen Zustand an die Stirnwand 28, die Zwischenwand 29 und die Oberwand 13 des Gehäuses an.

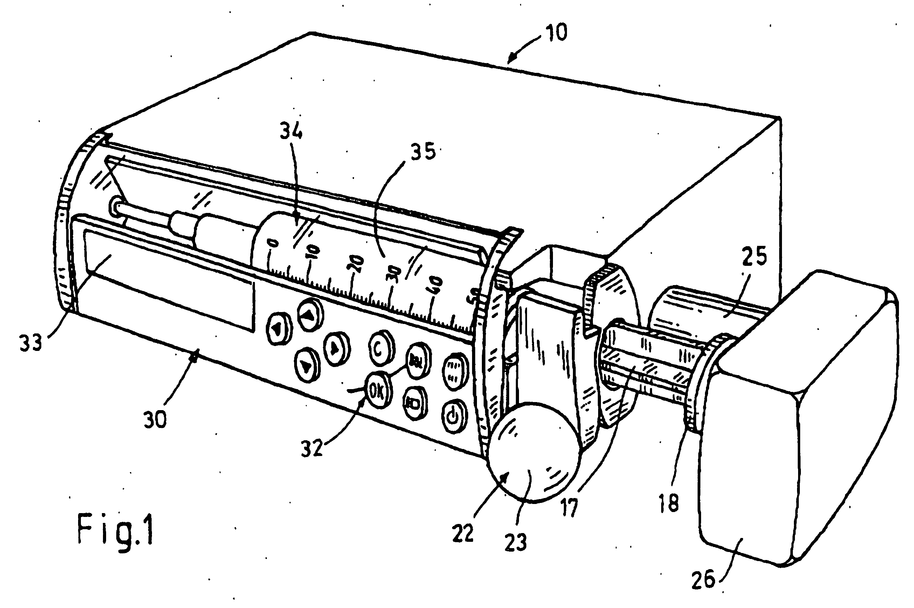

Wie Figur 1 zeigt, befindet sich an der Vorderseite des Deckels 30 das Bedienfeld 32, das verschiedene Tasten aufweist, die durch den Bediener zu betätigen sind. Außerdem befindet sich an der Vorderseite des Deckels eine Anzeigevorrichtung 33. Das Bedienfeld 32 und die Anzeigevorrichtung 33 sind elektrisch mit einem im Gehäuseinnern vorgesehenen Prozessor verbunden.

Das Bedienfeld 32 und die Anzeigevorrichtung 33 erstrecken sich nur über einen Teil der Höhe des Deckels 30. Im oberen Teil des Deckels ist ein transparentes Fenster 34 vorgesehen. Das Fenster 34 enthält eine Zylinderlinse, die eine Lupe 35 bildet. Bei geschlossenem Deckel vergrößert die Lupe 35 das Bild der Spritze 15 und insbesondere eine auf der Spritze befindliche Skala 36. Die Brennweite der Lupe ist größer als der Objektabstand a zur Lupe bei der kleinsten zu verwendenden Spritze.

Im Deckel 30 befindet sich eine Beleuchtungsvorrichtung 40, die im vorliegenden Fall drei Lichtquellen hat, welche Licht auf die Spritze 15 aussenden. Die Beleuchtungsvorrichtung 40 kann einerseits zum Beleuchten der Spritze dienen, zum anderen aber auch als. Signaleinrichtung, um im Falle eines Spritzenalarms die alarmgebende Spritzenpumpe zu kennzeichnen.

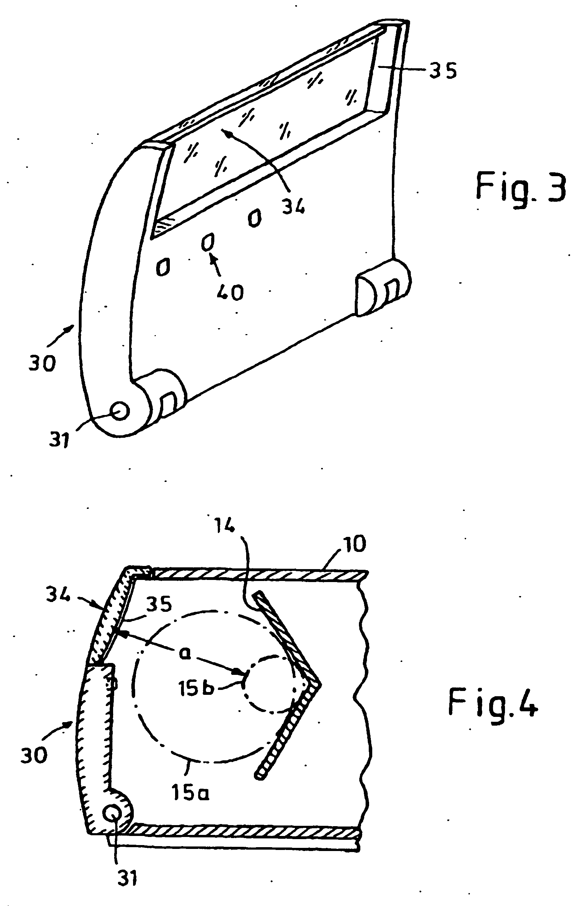

Figur 4 zeigt, dass Spritzen unterschiedlicher Größe in die Spritzenmulde 14 eingelegt werden können und dabei so ausgerichtet sind, dass sie durch die Lupe 35 hindurch betrachtet werden können. Mit 15a ist der Umriss einer großen Spritze bezeichnet und mit 15b der Umriss einer kleinen Spritze.

## Patentansprüche

1. Spritzenpumpe mit einem Gehäuse (10), das einen von einem Deckel (30) mit Fenster verschließbaren Raum für eine einzulegende Spritze (15) aufweist, einem relativ zu dem Gehäuse (10) linear bewegbaren Antriebskopf (26) zum Bewegen des Spritzenkolbens der Spritze, und einem Bedienfeld (32),
**dadurch gekennzeichnet,**
**dass** der Raum eine die Spritze (15) abstützende Spritzenmulde (14) enthält, dass der Deckel (30) die Spritzenmulde mindestens teilweise bedeckt und aufklappbar ist und, dass das Fenster (34) eine Lupe (35) zur vergrößerten Darstellung der Spritzenoberfläche aufweist, die die Spritzenoberfläche unterschiedlich großer Spritzen (15a, 15b), deren Durchmesser sich mindestens um den Faktor 2 unterscheiden, optisch vergrößert.

2. Spritzenpumpe nach Anspruch 1, **dadurch gekennzeichnet, dass** an dem Gehäuse ein Spritzenbügel (22) vorgesehen ist, der die Spritze (15) gegen die Spritzenmulde (14) drückt.

3. Spritzenpumpe nach Anspruch 1 oder 2, **dadurch gekennzeichnet, dass** die Lupe (35) eine langgestreckte Zylinderlinse aufweist.

4. Spritzenpumpe nach einem der Ansprüche 1-3 oder 2, **dadurch gekennzeichnet, dass** der Deckel (30) oder die Spritzenmulde (14) eine Beleuchtungsvorrichtung (40) zum Beleuchten der In der Spritzenmulde (14) befindlichen Spritze (15) aufweist.

5. Spritzenpumpe nach einem der Ansprüche 1-4, **dadurch gekennzeichnet, dass** der Deckel (30) ein Bedienfeld (32) und eine Anzeigevorrichtung (33) enthält.

## Revendications

1. Pompe seringue avec un boîtier (10) qui présente un espace pouvant être fermé par un couvercle (30) muni d'une fenêtre pour une seringue (15) à insérer, une tête d'entraînement (26) mobile de manière linéaire par rapport au boîtier (10) pour déplacer le piston de seringue de la seringue, et un ensemble de commande (32),
**caractérisée en ce que**
l'espace comprend un creux de seringue (14) supportant la seringue (15), le couvercle (30) recouvre le creux de seringue au moins partiellement et est rabattable, et la fenêtre (34) présente une loupe (35) pour une représentation agrandie de la surface de seringue, qui agrandit optiquement la surface de seringue de seringues (15a, 15b) de tailles différentes dont les diamètres diffèrent au moins du facteur 2.

2. Pompe seringue selon la revendication 1, **caractérisée en ce que** sur le boîtier un étrier de seringue (22) est prévu qui pousse la seringue (15) contre le creux de seringue (14).

3. Pompe seringue selon la revendication 1 ou 2, **caractérisée en ce que** la loupe (3.5) présente une lentille cylindrique allongée.

4. Pompe seringue selon l'une quelconque des revendications 1 à 3 ou 2, **caractérisée en ce que** le couvercle (30) ou le creux de seringue (14) présente un dispositif d'éclairage (40) pour éclairer la seringue (15) insérée dans le creux de seringue.

5. Pompe seringue selon l'une quelconque des revendications 1 à 4, **caractérisée en ce que** le couvercle (30) comprend un ensemble de commande (32) et un dispositif d'affichage (33).

## Claims

1. A syringe pump comprising a housing (10) including a space which is configured to accommodate a syringe (15) to be placed within the housing and is closable by a cover (30) provided with a window, a drive head (26) arranged for linear movement relative to the housing (10) for thus moving the syringe piston of the syringe, and an operating panel (32),
**characterized in**
**that** said space includes a syringe cavity (14) supporting the syringe (15), that the cover (30) at least partially covers the syringe cavity and is adapted to be hinged into an open position, and that the window (34) comprises a magnifying lens (35) generating an enlarged representation of the syringe surface for optical enlargement of the syringe surfaces of differently sized syringes (15a,15b) having diameters differing from each other at least by the factor 2.

2. The syringe pump according to claim 1, **characterized in that** the housing is provided with a syringe clamp (22) pressing the syringe (15) against the syringe cavity (14).

3. The syringe pump according to claim 1 or 2, **characterized in that** the magnifying lens (35) is an elongated cylinder lens.

4. The syringe pump according to any one of claims 1-3 or 2, **characterized in that** the cover (30) or the syringe cavity (14) is provided with an illumination means (40) for illuminating the syringe (15) arranged in the syringe cavity (14).

5. The syringe pump according to any one of claims 1-4, **characterized in that** the cover (30) includes an operating panel (32) and a display means (33).
